Europäisches Patentamt

European Patent Office

Office européen des brevets

(11) Publication number: **0 106 608**
**B1**

## (12) EUROPEAN PATENT SPECIFICATION

(45) Date of publication of patent specification: **06.12.89**

(21) Application number: **83305940.5**

(22) Date of filing: **30.09.83**

(51) Int. Cl.⁴: **A 61 K 37/04**

(54) Method of lyophilizing cold insoluble globulin.

(30) Priority: **07.10.82 JP 176917/82**

(43) Date of publication of application:
**25.04.84 Bulletin 84/17**

(45) Publication of the grant of the patent:
**06.12.89 Bulletin 89/49**

(84) Designated Contracting States:
**BE DE FR GB NL**

(56) References cited:
**EP-A-0 058 993**
**FR-A-2 301 266**

(73) Proprietor: **GREEN CROSS CORPORATION**
**15-1, Imabashi-1-chome Higashi-ku Osaka-shi**
**Osaka 541 (JP)**

(72) Inventor: **Ohmura, Takao**
**15-21, Aobadai 2-chome**
**Takarazuka-shi (JP)**
Inventor: **Hirao, Yutaka**
**14-1-606, Terauchi 2-chome**
**Toyonaka-shi (JP)**
Inventor: **Hanamura, Takuji**
**6-15, Hotarugaike Nakamachi 3-chome**
**Toyonaka-shi (JP)**
Inventor: **Ohmizu, Akimasa**
**4-10, Tomimatsu-2-chome**
**Amagasaki-shi Hyogo-ken (JP)**
Inventor: **Funakoshi, Satoshi**
**16-5, Aoyama 1-chome**
**Katano-shi (JP)**

(74) Representative: **Harrison, David Christopher**
**et al**
**MEWBURN ELLIS & CO 2/3 Cursitor Street**
**London EC4A 1BQ (GB)**

Courier Press, Leamington Spa, England.

# EP 0 106 608 B1

**Description**

This invention relates to a method of lyophilizing a cold insoluble globulin.

Cold insoluble globulin (hereinafter referred to as CIG) has in the past been called "large external trypsin sensitive protein (LETS)", "cell surface protein (CSP)", "cell adhesion factor (CAF)", "opsonic $\alpha_2$ surface binding glycoprotein (O-$\alpha_2$ SBG)" among other names. More recently it has generally been called CIG or fibronectin. It is a glycoprotein having a molecular weight of 440,000 which occurs in, besides plasma, mesenchymal cells such as fibroblasts or basilar membranes such as epidermis. Other known physicochemical properties of CIG include the mobility of $\alpha_2$ globulin, an isoelectric point of 5.0, a molecular extinction coefficient $A_1^{1\%}{}_{cm}$ 280 nm of 12.9—13.0, S 20, W of 11—14 S, and a carbohydrate content of 5%.

When blood coagulates, the bonding between γ-chains of fibrin is accelerated by the transglutaminase action of the blood coagulation factor XIII and cross-links of fibrin are formed. In this instance, cross-links between α-chains of fibrin are formed through CIG by the catalytic action of the same factor XIII, and thereby blood coagulation is enhanced. CIG also has the function of effecting adhesion or bonding between cells and between cell sustentacular tissues, and hence has the pharmacological effect of promoting wound healing of a trauma. Pharmacological effects of CIG hitherto reported include the treatment of septic shock and the treatment of infective disease by enhancing the opsonic action of phagocytes. Moreover, it is known that CIG has an anticancer or antileukemia effect due to its ability to enhance intercellular adhesion and necrotize cancer cells. Accordingly there are vast expectations for the clinical valve of CIG as a medicine.

When utilized as a medicine, CIG is preferably made up into lyophilized preparations because of its poor storage stability in solution. To keep the CIG stable to lyophilization, a neutral amino acid, monosaccharide, disaccharide, or sugar alcohol is added as a stabilizer. However, the use of these stabilizers is undesirable for providing CIG as a medicine, since the dissolution of the lyophilized preparation in distilled water for injection use takes a relatively long time and leaves fibrous insoluble matter, or turbidity develops.

In view of the above, the present inventors made extensive studies and have found that, when albumin together with at least one stabilizer selected from neutral amino acids, monosaccharides, disaccharides, and sugar alcohols is added to a CIG-containing aqueous solution before lyophilization, the time for dissolving the lyophilized preparations in distilled water for injection use is reduced, the insoluble matter and in consequence the turbidity do not develop, and additionally the stability of CIG to lyophilization is improved synergistically.

Therefore, the present invention provides a method of lyophilizing a CIG aqueous solution, in a concentration of 0.1 to 10% w/v, in the presence of albumin (0.01 to 5% w/v) and at least one stabilizer (1 to 10% w/v) selected from neutral amino acids, monosaccharides, disaccharides, and sugar alcohols, the amounts of albumin and the stabilizer being sufficient to prevent the occurrence of turbidity in an aqueous solution of the lyophilized CIG.

It is known that CIG is generally obtained by isolation from fractionated plasma protein, fibroblasts, or culture fluids of fibroblasts.

The method of this invention may be applied to CIG purified to a pharmaceutically acceptable degree and desirable contents of protein (including CIG) therein are 0.1—10% W/V. The purified CIG used in this invention may be prepared by any method, preferably one which includes heat-treatment for inactivating heptatitis virus. For instance, CIG obtained by a process of heating at 45°C—52°C to purify the CIG (as described in JP—A—121220/83) and heat treatment at 60°C for 10 hours to inactivate hepatitis virus (EP—A—0058993) is preferred for lyophilization by the method of this invention.

According to the heat treatment disclosed in EP—A—0058993, CIG which may have hepatitis virus activity can be virus-inactivated, while keeping at a minimum damage to the cold insoluble globulin, by heating its aqueous solution at 50 to 80°C for 5 to 20 hours in the presence of 10% (W/V) or more of at least one principal stabilizer chosen from neutral amino acids, monosaccharides, disaccharides, and sugar alcohols.

The stabilizer to be added to CIG in the present invention is the same as in EP—A—0058993, and may include stabilizers selected from, for example, glycine, alanine, valine, leucine, and isoleucine as neutral amino acids, i.e. monoaminomonocarboxylic acids; glucose, mannose, galactose, and fructors as monosaccharides; sucrose, maltose, and lactose as disaccharides; and mannitol, sorbitol, and xylitol as sugar alcohols. The amount of stabilizer used in the invention is however smaller, i.e. approximately 1—10% W/V. The stabilizer is added in combination with albumin, to prevent the occurrence of turbidity when the lyophilized CIG is dissolved in water.

The albumin to be added to the CIG is preferably prepared by purification from pooled plasma of normal human adults in a known way such as a fractionation with methanol, followed by treatment at 60°C for 10 hours to inactivate hepatitis virus. The amount of albumin to be added is in the order of 0.01—5% W/V, preferably 0.25—0.5% W/V, for the above purpose.

The symbol of "% W/V" means the concentration of a solute (g) in 100 ml of the solution, throughout the disclosure and claims.

2

The lyophilized preparation is usable for injection to a patient after being dissolved in distilled water. This invention is illustrated in more detail by the following Examples;.

Example

Hepatitis virus inactivated CIG obtained by the processes of JP—A—121220/83 and EP—A—0058993 was dialyzed against a phosphate-sodium chloride buffer solution. To the resulting solution were added 5% W/V of sucrose and 0.25% W/V of albumin and the CIG concentration was adjusted to 20 mg/ml. This solution was filtered to remove microorganisms, and 2-ml portions of the filtrate were placed separately in 10-ml tubes and lyophilized under drying conditions such that the temperature finally reached 30°C.

The moisture content in the lyophilized preparation was measured in accordance with the general testing method of "The Biological Preparation Standard" (issued by Saikin Seizai Kyokai—literally "Microorganism Preparation Association", July 16, 1979). The found moisture content was 0.2% by weight.

The lyophilized preparation, on adding 2 ml of distilled water for injection use, dissolved immediately, giving a colorless clear solution.

The CIG survival rate in this solution was determined by the single radial immunodiffusion method and the opsonic activity measuring method using fine hepatic pieces [Molnar, J., et al., Biochemistry, *18*, 3909 (1979)]. The values found by both the methods were no different from those before lyophilization.

Experimental Example

Experiments were conducted in order to confirm the stabilizing effect of this invention. In the experiments, specimens of the CIG invention prepared according to the methods of the above Experiment, after albumin and various stabilizers had been added either singly or in combination (the amounts added are shown Tables 1 and 2), were lyophilized and measured for the various items shown in the Tables.

The results indicated that; when only one of stabilizer(s) and albumin was added, the dissolution of the lyophilized preparations required considerable times and the resulting solutions were cloudy or contained fibrous insoluble matter (Table 1). When albumin was added in combination withfone of the various stabilizers, however the lyophilized preparations dissolved within one or several minutes and the resulting solutions were colorless and clear (Table 2).

TABLE 1

| Stabilizer | Concentration of stabilizer (W/V%) | CIG survival rate (%) | Opsonic activity survival rate (%) | Moisture content (%) | Finished state after lyophilization | Time for dissolving (min) | Appearance of solution |
|---|---|---|---|---|---|---|---|
| Mannitol | 2 | 88 | 81 | 1.0 | Good | 30 | Slightly cloudy |
| Glycine | 2.25 | 75 | 63 | 2.3 | Good | >30 | Cloudy |
| Sucrose | 5 | 100 | 92 | 0.2 | Good | 15 | Fibrous insoluble matter was observed |
| Glucose | 5 | 100 | 92 | 0.3 | Good | 115 | Fibrous insoluble matter was observed |
| Albumin | 1 | 100 | 90 | 1.2 | Good | 12 | Fibrous insoluble matter was observed |
| None | | 64 | 42 | 2.3 | Shrunken | Sparingly soluble | Cloudy |

EP 0 106 608 B1

TABLE 2

| Concentration of stabilizer (W/V%) | Concentration of albumin (W/V%) | CIG survival rate (%) | Opsonic activity survival rate (%) | Moisture content (%) | Finished state after lyophilization | Time for dissolving (min) | Appearance of solution |
|---|---|---|---|---|---|---|---|
| Sucrose (5) | 0.01 | 100 | 96 | 1.6 | Slightly shrunken | 10 | Fibrous insoluble matter was observed |
| | 0.05 | 100 | 100 | 0.9 | Good | 2—3 | Fibrous insoluble matter was observed |
| | 0.1 | 100 | 100 | 0.4 | Good | <1 | Colorless and clear |
| | 0.25 | 100 | 100 | 0.7 | Good | <1 | Colorless and clear |
| | 0.5 | 100 | 100 | 0.7 | Good | <1 | Colorless and clear |
| | 1.0 | 100 | 100 | 0.5 | Good | <1 | Colorless and clear |

TABLE 2 (continued)

| Concentration of stabilizer (W/V%) | Concentration of albumin (W/V%) | CIG survival rate (%) | Opsonic activity survival rate (%) | Moisture content (%) | Finished state after lyophilization | Time for dissolving (min) | Appearance of solution |
|---|---|---|---|---|---|---|---|
| Glucose (5) | 0.01 | 100 | 93 | 1.9 | Slightly shrunken | 10 | Fibrous insoluble matter was observed |
| | 0.05 | 100 | 100 | 0.6 | Good | 2—3 | Fibrous insoluble matter was observed |
| | 0.1 | 100 | 100 | 0.5 | Good | 2—3 | Fibrous insoluble matter was observed |
| | 0.25 | 100 | 100 | 0.2 | Good | <4 | Colorless and clear |
| | 0.5 | 100 | 100 | 0.8 | Good | <1 | Colorless and clear |
| | 1.0 | 100 | 100 | 0.6 | Good | <1 | Colorless and clear |

EP 0 106 608 B1

TABLE 2 (continued)

| Concentration of stabilizer (W/V%) | Concentration of albumin (W/V%) | CIG survival rate (%) | Opsonic activity survival rate (%) | Moisture content (%) | Finished state after lyophilization | Time for dissolving (min) | Appearance of solution |
|---|---|---|---|---|---|---|---|
| Glycine (2.25) | 0.01 | 72 | 55 | 1.8 | Slightly shrunken | 30 | Cloudy |
| | 0.05 | 81 | 62 | 0.8 | Slightly shrunken | 20 | Cloudy |
| | 0.1 | 95 | 82 | 1.8 | Good | 2—3 | Slightly cloudy |
| | 0.25 | 100 | 85 | 1.3 | Good | 2—3 | Fibrous insoluble matter was observed |
| | 0.5 | 100 | 90 | 0.6 | Good | 2—3 | Colorless and clear |
| | 1.0 | 100 | 90 | 0.9 | Good | 2—3 | Colorless and clear |
| Mannitol (2) | 0.01 | 94 | 86 | 0.7 | Slightly shrunken | 20 | Slightly cloudy |

TABLE 2 (continued)

| Concentration of stabilizer (W/V%) | Concentration of albumin (W/V%) | CIG survival rate (%) | Opsonic activity survival rate (%) | Moisture content (%) | Finished state after lyophilization | Time for dissolving (min) | Appearance of solution |
|---|---|---|---|---|---|---|---|
| Mannitol (2) | 0.05 | 100 | 95 | 1.8 | Good | 10 | Fibrous insoluble matter was observed |
| | 0.1 | 100 | 95 | 1.5 | Good | 2—3 | Fibrous insoluble matter was observed |
| | 0.25 | 100 | 100 | 0.8 | Good | 2—3 | Colorless and clear |
| | 0.5 | 100 | 100 | 0.5 | Good | <1 | Colorless and clear |
| | 1.0 | 100 | 100 | 0.5 | Good | <1 | Colorless and clear |

**Claims**

1. A method of lyophilizing an aqueous solution of a cold insoluble globulin in a concentration of 0.1 to 10% w/v, characterised by lyophilizing the solution in the presence of 0.01 to 5% w/v of albumin and 1 to 10% w/v of at least one stabilizer selected from neutral amino acids, monosaccharides, disaccharides, and sugar alcohols, to prevent the occurrence of turbidity when the lyophilized cold insoluble globulin is dissolved in water.

2. A method according to claim 1, wherein the neutral amino acid is used and is glycine, alanine, valine, leucine or isoleucine.

3. A method according to claim 1, wherein the monosaccharide is used and is glucose, mannose, galactose or fructose.

4. A method according to claim 1, wherein the disaccharide is used and is sucrose, maltose or lactose.

5. A method according to claim 1, wherein the sugar alcohol is used and is mannitol, sorbitol or xylitol.

6. A method according to any one of claims 1 to 5 wherein the albumin is used in an amount of from 0.25 to 0.5% w/v.

7. A lyophilized cold insoluble globulin composition which contains a cold insoluble globulin, albumin and at least one stabilizer selected from neutral amino acids, monosaccharides, disaccharides and sugar alcohols.

**Patentansprüche**

1. Verfahren zur Lyophilisierung einer wäßrigen Lösung eines in der Kälte unlöslichen Globulins in einer Konzentration von 0,1 bis 10% Gew./Vol., gekennzeichnet durch Lyophilisierung der Lösung in Gegenwart von 0,01 bis 5% Gew./Vol. Albumin und 1 bis 10% Gew./Vol. mindestens eines Stabilisators, ausgewählt aus neutralen Aminosäuren, Monosacchariden, Disacchariden und Zuckeralkoholen, zur Verhinderung des Auftretens von Trübung, wenn das lyophilisierte in der Kälte unlösliche Globulin in Wasser gelöst wird.

2. Verfahren nach Anspruch 1, wobei die neutrale Aminosäure verwendet wird und diese Glycin, Alanin, Valin, Leucin oder Isoleucin ist.

3. Verfahren nach Anspruch 1, wobei das Monosaccharid verwendet wird und dieses Glukose, Mannose, Galactose oder Fructose ist.

4. Verfahren nach Anspruch 1, wobei das Disaccharid verwendet wird und dieses Sucrose, Maltose oder Lactose ist.

5. Verfahren nach Anspruch 1, wobei der Zuckeralkohol verwendet wird und dieser Mannit, Sorbit oder Xylit ist.

6. Verfahren nach einem der Ansprüche 1 bis 5, wobei das Albumin in einer Menge von 0,25 bis 0,5% Gew./Vol. verwendet wird.

7. Lyophilisierte, in der Kälte unlösliche Globulin-Zusammensetzung, welche ein in der Kälte unlösliches Globulin, Albumin und mindestens einen Stabilisator enthält, welcher ausgewählt ist aus neutralen Aminosäuren, Monosacchariden, Disacchariden und Zuckeralkoholen.

**Revendications**

1. Procédé de lyophilisation d'une solution aqueuse d'une globuline insoluble à froid, selon une concentration de 0,1 à 10% poids/volume, caractérisé en ce qu'on lyophilise la solution en présence de 0,01 à 5% poids/volume d'albumine, et de 1 à 10% poids/volume d'au moins un stabilisant choisi parmi les aminoacides neutres, les monosaccharides, les disaccharides et les alcools dérivés de sucre, pour empêcher l'apparition de turbidité, lorsque la globuline insoluble à froid lyophilisée est dissoute dans l'eau.

2. Procédé selon la revendication 1, dans lequel on utilise un aminoacide neutre choisi parmi la glycine, l'alanine, la valine, la leucine et l'isoleucine.

3. Procédé selon la revendication 1, dans lequel on utilise en monosaccharide choisi parmi le glucose, le mannose, le galactose et le fructose.

4. Procédé selon la revendication 1, dans lequel on utilise un disaccharide choisi parmi le saccharose, le maltose et le lactose.

5. Procédé selon la revendication 1, dans lequel on utilise un alcool dérivé de sucre, choisi parmi le mannitol, le sorbitol et le xylitol.

6. Procédé selon l'une quelconque des revendications 1 à 5, dans lequel on utilise de l'albumine selon une quantité de 0,25 à 0,5% poids/volume.

7. Composition lyophilisée à base de globuline insoluble à froid, contenant une globuline insoluble à froid, de l'albumine et au moins un stabilisant choisi parmi les aminoacides neutres, les monosaccharides, les disaccharides et les alcools dérivés de sucre.